# EUROPEAN PATENT SPECIFICATION

(11) **EP 0 815 803 B1**
(45) Date of publication and mention of the grant of the patent: **08.10.2003**
(21) Application number: 97202017.6
(22) Date of filing: 02.07.1997
(51) Int. Cl.: A61F 2/01

(54) **Catheter with temporary vena cava filter**
Katheter mit provisorischem vena-cava-Filter
catheter a filtre provisoire pour la veine cave

(30) Priority: 03.07.1996 NL 1003497
(43) Date of publication of application: 07.01.1998
(73) Proprietor: CORDIS EUROPA N.V., NL-9301 LJ Roden (NL)
(72) Inventor: Homsma, Tjeerd, 9302 ER Roden (NL); Boudewijn, Alexander Christiaan, 9351 KS Leek (NL)
(74) Representative: 't Jong, Bastiaan Jacob

(56) References cited:
- EP-A- 0 437 121
- FR-A- 2 606 642
- FR-A- 2 652 267
- FR-A- 2 696 092
- US-A- 3 952 747

## Description

The invention relates to a catheter with a temporary vena cava filter which comprises a tube-like basic body with a proximal end and a distal end. A filter element has been connected to the basic body which comprises a number of pliable strips arranged at equal distances from one another around the circumference of the basic body, which are connected with opposite ends to a front and rear supporting member respectively. The supporting members are movable in relation to one another so that they can be moved towards and away from each other. In the state in which have been moved away from each other the pliable strips are straightened and are positioned against the basic body of the catheter, so that the latter can be introduced into the body of a patient. As soon as the filter element has been manoeuvred into the required position inside the vena cava, the supporting members are moved towards each other by means of expanding means, as a result of which the pliable strips bend outwards until they make contact with the wall of the vena cava. The strips bending outwards consequently form a filter which may be used to retain blood thrombi.

Such a catheter with a vena cava filter is typically left for some time inside the body of the patient. During that time the tunica intima of the vena cava may grow around the strips with which it makes contact, so that in principle the filter cannot be removed any more without damaging the tunica intima.

The object of the invention is therefore to provide a catheter with a temporary vena cava filter, which does not have this disadvantage.

This aim is achieved with a catheter according to claim 1. By moving the engaging means from the engaging position into the disengaging position, it is as a result possible to disengage the strip ends without any force being applied to the strips themselves. Damage to the tunica intima which has grown around the strips is thus prevented in a reliable manner.

With the state-of-the-art catheter the damage is due to the fact that the strip ends have to be detached by applying an axial tensile force to the strip ends. This axial tensile force results in an inward axial force acting on the bent sections of the strips, as a result of which the strips may be torn free form the tunica intima. As, with the catheter according to the invention, no tensile force is applied at all to the strips, no damage whatsoever is done when removing the catheter. A device having the features of the preamble of claim 1 is disclosed by document FR-A-2 652 267.

A suitable embodiment of the catheter according to the invention has been characterised in claim 2. After removing the blocking means, the strip ends can move freely out of the mounting without any significant axial force being applied.

Additionally, the measure as set out in claim 3 is preferably employed. The blocking means can in that case act immediately on the strip ends.

With the measure as set out in claim 4 it is achieved that on moving the blocking means from the engaging position into the disengaging position, the strip end is pulled along outwards, out of the mounting, so that it is disengaged positively.

By employing the measure as set out in claim 5 only limited forces need to be applied to the blocking means.

Another suitable embodiment has been characterised in claim 6. In the engaging state the larger cross-section towards the ends of the strips cannot pass the smaller cross-section of the space not occupied by the blocking means, so that the strip ends are enclosed directly inside the supporting member. By pulling back the blocking means, a greater space is created from where the strip ends can move freely outwards.

In order to prevent undesired disengaging of the strip ends, the measure as set out in claim 7 is preferably employed.

A very suitable and easy to manipulate embodiment has been characterised in claim 8. During the introduction and expanding of the filter element, the central tube-like body is kept immobile in relation to the tube-like body, so that the engaging means remain in the engaging position. The outer tube-like body can be moved, in relation to the central and the inner tube-like body, in a distal direction for the purpose of expanding the pliable strips. The central tube-like body can be moved in relation to the inner tube-like body for the purpose of moving the engaging means into the disengaging position. By manufacturing the catheter according to a further preferred feature of the invention in such a way that the strip ends are connected to the front supporting member in a detachable manner, the engaging means can be moved easily into the disengaging position by moving the central tube-like body in relation to the inner tube-like body inside the proximal body.

The invention will be explained in greater detail in the following description with reference to examples of embodiments illustrated in the figures.

Figure 1 shows a section of a catheter according to the invention carrying the filter element after having been positioned for some time inside the vena cava illustrated here in a cut away view.

Figure 2 shows the catheter of figure 1 in a partial longitudinal cross-section.

Figure 3 shows a view corresponding to figure 2 whilst disengaging the strip ends.

Figure 4 shows a view corresponding to the figures 2 and 3 after disengaging the strip ends.

Figures 5 - 11 show cross-sections of variations of embodiments of the catheter according to the invention at the location of the supporting member to which the strips have been connected in a detachable manner.

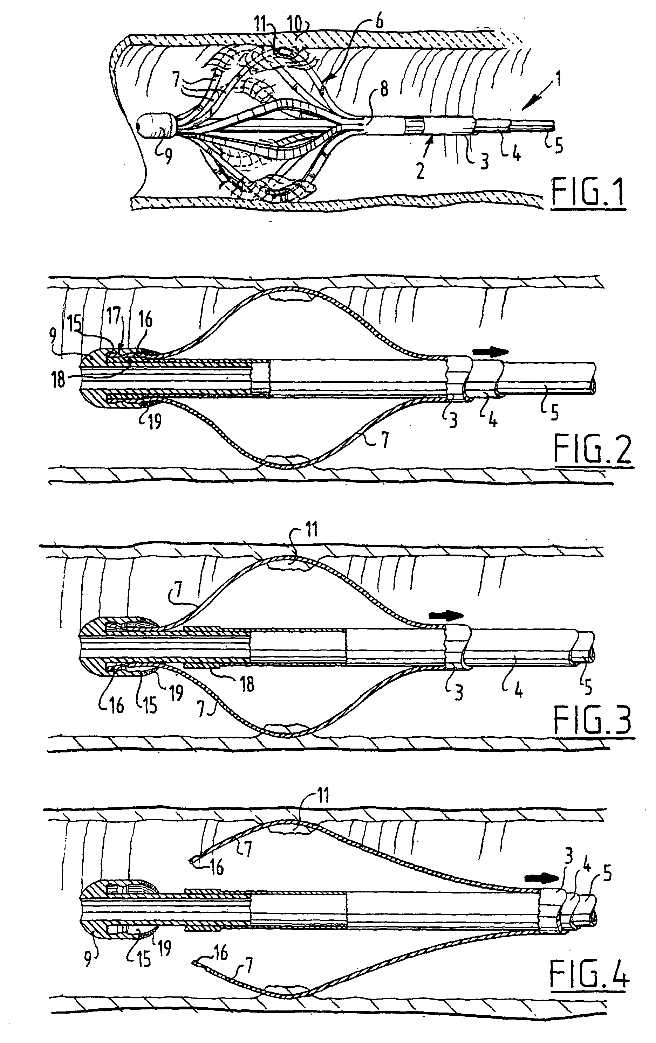

In figure 1 only the distal end-section with the filter element 6 of the catheter 1 has been illustrated.

The catheter 1 comprises a tube-like basic body 2 which comprises in the case of this preferred embodiment an outer tube-like element 3, a central tube-like element 4 and an inner tube-like element 5. The elements 4 and 5 respectively have been received inside the lumens of the elements 3 and 4. As will be described in greater detail below, the tube-like elements are movable in relation to one another in a longitudinal direction.

The filter element 6 comprises a number of pliable strips 7 arranged at equal distances from each other around the circumference of the basic body 2. The strips 7 have opposite ends which are connected to the front supporting member 9 and the rear supporting member 8 respectively. With the embodiment shown here, the strips 7 have been formed by wall sections of the outer tube-like element 3, so that in this case the supporting member 8 can be considered to form an integrated part of the outer tube-like element 3 of the basic body 2.

As can be seen in the figures 2 - 4, the front supporting member 9 is connected to the inner tube-like element 5. Consequently the supporting members 8 and 9 can be moved in relation to one another both towards each other and away from each other by moving the tube-like elements 3 and 5 in the longitudinal direction in relation to one another. When the supporting members 8 and 9 are moved away from one another the strips 7 will be straightened, and when the supporting members 8 and 9 are moved towards each other the strips 7 will bend outwards as shown in the figures 1 - 4.

It will be clear that the catheter 1 will be introduced into a patient in the state wherein the supporting members 8 and 9 are kept at a distance from one another in order to straighten the strips, so that a minimal cross-section of the catheter at the location of the filter element 6 is obtained.

When the filter element 6 has been arranged in the required position inside the vena cava of the patient, the inner tube-like element 5 is moved in a proximal direction in relation to the outer tube-like element 3, so,that the strips 7 will come into contact with the wall of the vena cava 10. Thus the strips bent outwards form a filter element which can retain blood thrombi et cetera.

As has been indicated in the figures at number 11, the tunica intima of the vena cava 10 is arranged around the strips 7 positioned against the wall of the vena cava, so that they are enclosed thereby. When removing the catheter it will consequently not suffice to straighten the strips 7 again, as this would result in damage to the vena cava.

With the embodiment shown, the ends of the strips 7 which are connected to the supporting member 9 have been made detachable with the embodiment shown in order to prevent such damage. The ends 16 of the strips 7 have been fixed by detachable engaging means in the supporting member 9. To this end the supporting member 9 comprises a mounting or cavity 15 which has a radial width which is such that the strip end 16 can move itself in and out of it freely. The radial outer wall of the mounting 15 has been provided with a bulge and the strip end 16 with a matching indentation. The blocking means 18, which are formed in the example of this embodiment by a cylindrical tube section, extend inside the mounting 15 and leave a space in the mounting 15 which has a shape which is complementary to that of the strip end 16. In the engaging position of the blocking means 18 illustrated in figure 2, in which the latter extend inside the mounting 15, the strip end 16 has been fixed to the supporting member 9, as the profile of the indentation and the bulge prevent unimpeded outward movement of the strip end 16 .

When the catheter has to be removed, the strip ends 16 have to be disengaged. This is done by moving the blocking means 18, which, as has been mentioned above, are connected to the inner tube-like element 4, to the right in relation to the supporting member 9 and consequently in relation to the inner tube-like element 5. As a result enough space is created inside the mounting 15 that the strip end 16 can move out of the mounting 15 unhindered. As can be seen in figure 4, the outer tube-like body 3 is subsequently moved in relation to the inner tube-like body 5 in the proximal direction, as a result of which, without any significant tensile force been applied, the strips 7 are released from the mounting 15 and the catheter with the filter element can be withdrawn. On pulling back, the strips slide through the tissue 11 which has grown around them without damaging the tissue.

As, with reference to the figures, will be clear from the above, fitting the strip end 16 to the supporting member 9 is based on the fact that the strip end comprises a section 17 with a cross-section which decreases in the direction away from the end, that is to say towards the right as seen in the figure, and that the blocking means 18 in the engaging position leave space inside the mounting 15 the shape of which is complementary to the section 17.

As furthermore can be seen in the figures, the front supporting end 9 has been provided at its relatively proximal end with a pliable lip 19, which lies in a resilient manner against the strips when they are fixed. Thus a smooth transition is achieved between the supporting member 9 and the strips.

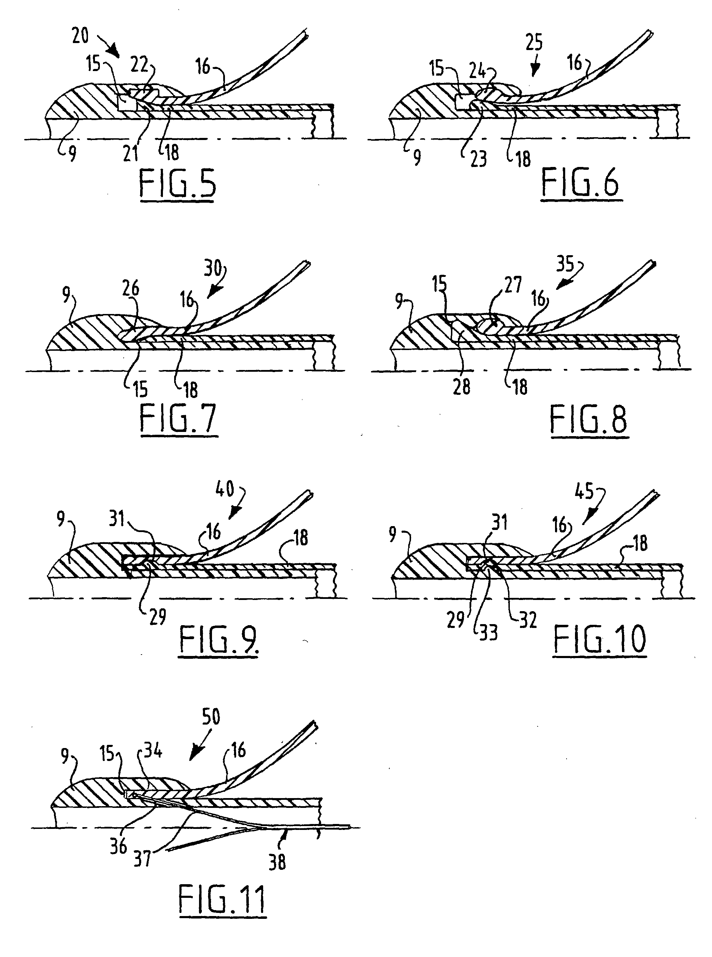

The engaging means can be embodied in many different ways. The figures 5 - 11 show a few possibilities.

With the embodiment of the catheter 20 as shown in figure 5, the strip end has been provided with a projection 22 which engages in a groove inside the mounting 15 of the supporting member. In the engaging position moved to the right, the blocking means 18 comprise a wedge-shaped end 21 which keeps the projection 22 pushed into the groove. In order to disconnect, the blocking means 18 are moved, as seen in the figure, towards the left, that is to say, are pushed further into the mounting 15, as a result of which the projection 22 can be released from the groove.

The embodiment of the catheter 25 as shown in figure 6 substantially corresponds to the one shown in figure 5. Also in this case the strip end 16 has been provided with a projection 24 which engages in an indentation of a wall of the mounting 15. The blocking means 18 have been provided at their relatively distal ends with a bulge 23 with which the strip end is kept pushed into the indentation. For the purpose of releasing the strip ends the blocking means 18 are moved to the left, as a result of which sufficient space is created to release the strip end.

With the catheter 30 of figure 7 the strip end 16 has also been provided with a bulge 26 which is pushed into a mounting 15 of the supporting member. In this case the blocking means 18 form a simple sleeve which narrows the 'exit' of the mounting 15, as a result of which the bulge 26 cannot move out of the mounting 15 as long as the blocking means 18 are in the engaging position illustrated. By simply moving the blocking means 18 to the right, sufficient space is created for the bulge 26 to pass and for the release of the strip end 16.

The catheter 35 as shown in figure 8 comprises engaging means which are based on a somewhat different principle. The strip end 16 has in this case been provided with a bulge 27 which slots into an indentation in the mounting of the supporting member. The blocking means 18 have however been provided with a rather pronounced wedge-shaped end 28 which is associated with a complementary surface of the mounting 15. In the state illustrated in figure 8 both the strip end 16 and the blocking means 18 have been fixed. In order to release the strip end 16, the blocking means 18 are moved over a short distance to the right, as a result of which the wedge-shaped end 28 pushes the section of the supporting member 9 adjoining the mounting 15 outwards, so that the bulge 27 can move out.

With the catheter 40 of figure 9 the blocking means 18 and the strip end 16 are provided with profiles which engage at right angles to the displacement direction extending from left to right of the blocking means 18. These profiles comprise a bulge 29 arranged on the blocking means 18 and a corresponding indentation arranged in the strip end 31. When the strip end 16 and the blocking means 18 have been inserted into the mounting as shown in figure 9, the strip end 16 is fixed in relation to the supporting member 9. Together with the blocking means 18, the strip end 16 can be moved freely inside the mounting so that, when the blocking means 18 are moved to the right from the engaging position shown into the disengaging position, the strip end 16 moves along and is consequently moved out of the mounting together with the blocking means 18.

With the embodiment of the catheter 45 as shown in figure 10, the blocking means 18 have additionally been provided with an indentation 32 and the inner wall of the mounting with a corresponding bulge 33. As a result the blocking means 18 are retained together with the strip end 16 in the mounting of the supporting member 9 by means of snap action. When moving the blocking means 18 from the engaging position illustrated into the disengaging position, a certain resistance has to be overcome in order to pass the bulge 33. Subsequently the blocking means 18 move the strip end 16 outwards, free of the supporting member 9.

Finally, a catheter 50 has been shown in figure 11, comprising engaging means working according to yet another principle. The strip end 16 has also in this case been inserted into a mounting of the supporting member 9. In this case the engaging means comprise an oblique bore 34 in the strip end 16 and, in the inserted state of the strip end 16, a bore 36 in the supporting member 9, positioned in line with this bore 34. A stiff wire 37 has been introduced into these aligned bores 34 and 36. This wire 37 prevents the strip end 16 from moving outwards, out of the mounting 15.

For the purpose of disengaging the strip end 16, the catheter 50 has been provided with a pull wire 38 extending towards the proximal end, which has been connected to the blocking wire 37. By pulling at the pull wire 38 at the proximal end, the blocking wire 37 is moved form the engaging position illustrated into the disengaging position, in which it has been withdrawn from at least the oblique bore 34 in the strip end 16. The strip end 16 can then move freely outwards, out of the mounting 15.

It will be clear that the invention can be given many different types of embodiments. Although in the variations of embodiments the strip end has been received each time in a mounting of the supporting member concerned, the scope of the invention is defined by the appended claims.

## Claims

1. Catheter (1) with a temporary vena cava filter comprising a tube-like basic body (2) with a proximal end and a distal end, a filter element (6) comprising a front supporting member (9) connected to the basic body and, positioned at a distance thereof in the direction of the proximal end, a rear supporting member (8) connected to the basic body, a number of pliable strips (7) arranged at equal distances from one another around the circumference of the basic body each comprising opposite ends which are connected to the front and rear supporting member respectively and expanding means which can move at least one of the supporting members towards and away from the other supporting member for the purpose of bending or straightening the pliable strips and can be controlled at the proximal end, **characterised in that** the pliable strips are connected to at least one of the supporting members by detachable engaging means which can be moved between an engaging position in which they fix the strip end to the supporting member and a disengaging position in which they leave the strip end free and wherein the engaging means are associated with control means arranged close to the proximal end for the purpose of moving the engaging means form the engaging position into the disengaging position.

2. Catheter as claimed in claim 1, wherein the engaging means comprise for each strip a mounting (15) formed at the supporting member for the purpose of loosely receiving the strip end and separate blocking means (18) which can be moved by the control means for the purpose of fixing the strip end in the mounting.

3. Catheter as claimed in claim 2, wherein the blocking means extend inside the mounting in the engaging position.

4. Catheter as claimed in claim 3, wherein the blocking means and the strip end have been provided with profiles engaging at right angles to the displacement direction of the blocking means.

5. Catheter as claimed in claim 4, wherein the strip end, together with the blocking means, can move freely inside the mounting.

6. Catheter as claimed in claim 4, wherein the strip end comprises a section which has a cross-section which reduces in the direction away from the end, and the blocking means in the engaging position leave a space clear in the mounting the shape of which is complementary to this section.

7. Catheter as claimed in claim 2, provided with snapping means (32, 33) for the purpose of retaining the blocking means in the engaging position.

8. Catheter as claimed in claim 1, wherein the basic body comprises an outer tube-like body (3) with a lumen, extending through this lumen a central tube-like body (4) also comprising a lumen and extending through this lumen an inner tube-like body (5), wherein the front supporting member (9) is connected to the inner tube-like body (5), the rear supporting member (8) to the outer tube-like body (3) and the engaging means to the central tube-like body (4) and wherein the tube-like bodies are connected to control means at the proximal end for the purpose of moving the tube-like bodies in relation to one another.

9. Catheter as claimed in claim 8, wherein the strip ends are connected to the front supporting member in a detachable manner.

## Patentansprüche

1. Katheter (1) mit einem temporären Vena-cava-Filter, welcher einen röhrenartigen Grundkörper (2) mit einem proximalen Ende und einem distalen Ende umfasst, sowie ein Filterelement (6), welches ein vorderes, mit dem Grundkörper verbundenes Trageteil umfasst sowie, in einem gewissen Abstand in Richtung des proximalen Endes befindlich, ein hinteres, mit dem Grundkörper verbundenes Trageteil (8), eine Anzahl von biegsamen, äquidistant voneinander um den Umfang des Grundkörpers herum angeordneter Leisten (7), von denen jede entgegengesetzte Enden umfasst, welche mit dem vorderen beziehungsweise hinteren Trageteil verbunden sind,-und Ausdehnungsmittel, welche mindestens eines der Trageteile auf das anderen zu und von ihm weg bewegen können, um die biegsamen Leisten zu krümmen oder zu strecken, und welche am proximalen Ende gesteuert werden können, **dadurch gekennzeichnet, dass** die biegsamen Leisten mit mindestens einem der Trageteile durch abnehmbare Eingriffsmittel verbunden sind, welche zwischen einer Eingriffsposition, in der sie das Leistenende an dem Trageteil fixieren, und einer Freigabeposition, in der sie das Leistenende freigeben, bewegt werden können, wobei die Eingriffsmittel mit Steuermitteln in Verbindung stehen, welche zum Zweck der Bewegung der Eingriffsmittel von der Eingriffsposition in die Freigabeposition nahe dem proximalen Ende angeordnet sind.

2. Katheter nach Anspruch 1, bei welchem die Eingriffsmittel für jede Leiste eine am Trageteil gebildete Fassung (15) umfassen, um das Leistenende lose aufzunehmen, sowie separate Sperrmittel (18), welche durch die Steuermittel bewegt werden können, um das Leistenende in der Fassung zu fixieren.

3. Katheter nach Anspruch 2, bei welchem die Sperrmittel in der Eingriffsposition in die Fassung hineinreichen.

4. Katheter nach Anspruch 3, bei welchem die Sperrmittel und das Leistenende Profile aufweisen, welche unter rechtem Winkel zur Versatzrichtung der Sperrmittel ineinander einrasten.

5. Katheter nach Anspruch 4, bei welchem sich die Leistenenden zusammen mit den Sperrmitteln frei innerhalb der Fassung bewegen können.

6. Katheter nach Anspruch 4, bei welchem das Leistenende einen Abschnitt umfasst, dessen Durchmesser mit der Entfernung vom Ende abnimmt, und bei welchem die Sperrmittel in der Eingriffsposition Raum in der Fassung frei lassen, dessen Gestalt zu der dieses Abschnitts komplementär ist.

7. Katheter nach Anspruch 2, welcher Schnappmittel (32, 33) aufweist, um die Sperrmittel in der Eingriffsposition zu halten.

8. Katheter nach Anspruch 1, bei welchem der Grundkörper einen äußeren röhrenartigen Körper (3) mit einem Hohlraum umfasst, durch den sich ein zentraler röhrenartiger Körper (4) erstreckt, welcher ebenfalls einen Hohlraum umfaßt, durch den sich ein innerer röhrenartiger Körper (5) erstreckt, wobei das vordere Trageteil (9) mit dem inneren röhrenartigen Körper (5), das hintere Trageteil (8) mit dem äußeren röhrenartigen Körper (3) und die Eingriffsmittel mit dem zentralen röhrenartigen Körper (4) verbunden sind, und wobei die röhrenartigen Körper am proximalen Ende mit Steuermitteln verbunden sind, um die röhrenartigen Körper relativ zueinander zu bewegen.

9. Katheter nach Anspruch 8, wobei die Leistenenden in abnehmbarer Weise mit dem vorderen Trageteil verbunden sind.

## Revendications

1. Cathéter (1) avec un filtre provisoire pour veine cave comprenant un corps de base en forme de tube (2) avec une extrémité proximale et une extrémité distale, un élément formant filtre (6) comprenant un membre support avant (9) relié au corps de base et, placé à une certaine distance de là en direction de l'extrémité proximale, un membre support arrière (8) relié au corps de base, plusieurs bandes pliables (7) disposées à équidistance les unes des autres autour de la circonférence du corps de base chacune comprenant des extrémités opposées qui sont reliées au membre support avant et au membre support arrière respectivement et un dispositif d'extension qui peut rapprocher ou éloigner au moins l'un des membres support de l'autre membre support en vue de plier ou d'étirer les bandes pliables et peut être contrôlé au niveau de l'extrémité proximale, **caractérisé en ce que** les bandes pliables sont fixées à au moins l'un des membres support par un dispositif d'engagement amovible qui peut être déplacé entre une position d'engagement dans laquelle il fixe l'extrémité de la bande au membre support et une position de dégagement dans laquelle il libère l'extrémité de la bande, et dans lequel le dispositif d'engagement est associé avec un dispositif de contrôle disposé près de l'extrémité proximale en vue de passer le dispositif d'engagement de la position d'engagement à la position de dégagement.

2. Cathéter selon la revendication 1, dans lequel le dispositif d'engagement comprend pour chaque bande un montage (15) formé au niveau du membre support en vue de recevoir de façon lâche l'extrémité de la bande et un dispositif de blocage séparé (18) qui peut être déplacé par le dispositif de contrôle en vue de fixer l'extrémité de la bande dans le montage.

3. Cathéter selon la revendication 2, dans lequel le dispositif de blocage se prolonge à l'intérieur du montage dans la position d'engagement.

4. Cathéter selon la revendication 3, dans lequel le dispositif de blocage et l'extrémité de la bande ont été munis de profiles s'engageant à angle droit de la direction de déplacement du dispositif de blocage.

5. Cathéter selon la revendication 4, dans lequel l'extrémité de la bande, avec le dispositif de blocage, peut se déplacer librement à l'intérieur du montage.

6. Cathéter selon la revendication 4, dans lequel l'extrémité de la bande comprend un tronçon ayant une section transversale qui se réduit en s'éloignant de l'extrémité, et le dispositif de blocage dans la position d'engagement laisse un espace vide dans le montage dont la forme est complémentaire de cette section.

7. Cathéter selon la revendication 2, muni d'un dispositif d'encliquetage (32,33) en vue de maintenir le dispositif de blocage dans la position d'engagement.

8. Cathéter selon la revendication 1, dans lequel le corps de base comprend un corps externe en forme de tube (3) avec une lumière, un corps central en forme de tube (4) qui s'étend au travers de cette lumière et comprend aussi une lumière et un corps interne en forme de tube (5) qui s'étend au travers de cette lumière, dans lequel le membre support avant (9) est relié au corps interne en forme de tube (5), le membre support arrière (8) est relié au corps externe en forme de tube (3) et le dispositif d'engagement est relié au corps central en forme de tube (4) et dans lequel les corps en forme de tube sont reliés au dispositif de contrôle au niveau de l'extrémité proximale en vue de déplacer les corps en forme de tube en relation les uns avec autres.

9. Cathéter selon la revendication 8, dans lequel les extrémités des bandes sont reliées au membre support avant de façon amovible.
